# EUROPEAN PATENT SPECIFICATION

(11) **EP 4 355 241 B1**
(45) Date of publication and mention of the grant of the patent: **26.08.2026**
(21) Application number: 22736007.0
(22) Date of filing: 14.06.2022
(51) Int. Cl.: A61B 34/00, A61B 5/00, A61B 5/06, A61B 34/20

(54) **ENDOSCOPIC MAGNETIC GUIDANCE SYSTEM**
ENDOSKOPISCHES MAGNETISCHES FÜHRUNGSSYSTEM
SYSTÈME DE GUIDAGE MAGNÉTIQUE ENDOSCOPIQUE

(30) Priority: 14.06.2021 US 202163210329 P; 31.05.2022 US 202217828432
(43) Date of publication of application: 24.04.2024
(73) Proprietor: Ethicon, Inc, Raritan, NJ 08869 (US)
(72) Inventor: KUHN, Matthew, Houston, Texas 77021 (US)
(74) Representative: Carpmaels & Ransford LLP
(86) International application number: PCT/IB2022/055474
(87) International publication number: WO 2022/264013

(56) References cited:
- EP-A2- 2 255 743
- WO-A1-2013/073710
- US-A1- 2004 064 153
- US-A1- 2015 243 425

## Description

### CROSS-REFERENCE TO RELATED APPLICATIONS

This application claims priority to and the benefit of United States (US) Patent Application Number 63/210,329 filed June 14, 2021.

### BACKGROUND

Significant progress has been made in automating the navigation of medical devices in the body. Remote navigation systems allow a physician to remotely orient the distal end of a medical device in the body. In recent years, magnetically navigable and controllable catheters have been used. These catheters have allowed more aspects of ablation and mapping procedures to be automated for improved accuracy and efficiency. They also provide the benefit of lowering radiation exposure at least for the treating physicians by enabling catheter control from a remote location away from the patient. These devices typically house a position sensor and a sufficient volume of magnetic or magnetizable material to enable suitable magnetic response to an external magnetic guidance system.

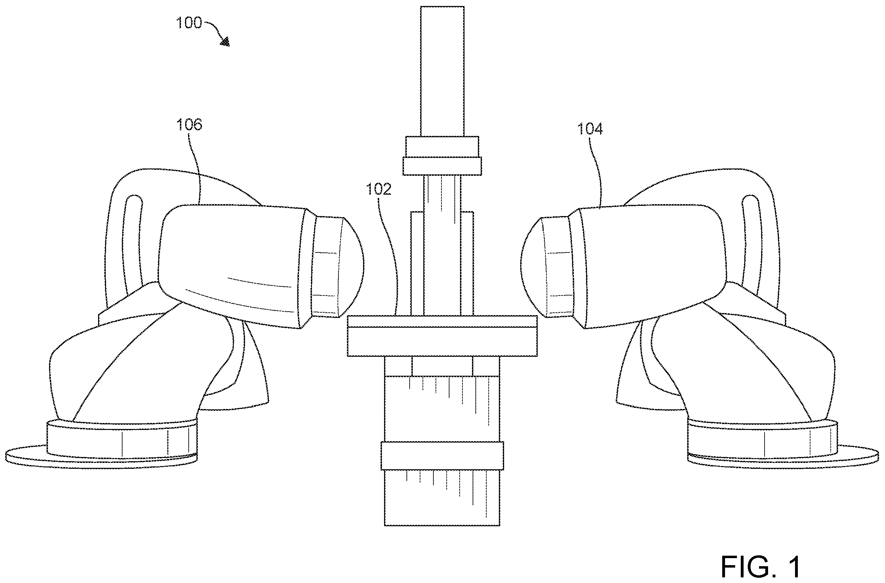

A magnetic guidance system 100, as shown in FIG. 1 for example, typically utilizes two large permanent magnets 104 and 106 mounted on either side of the patient table 102 that generate magnetic navigation fields. The magnets allow for controlling the position and orientation of magnet devices such as catheters introduced into the body. As seen in FIG. 1, these systems are large, space encumbering, comprise many complex electromechanical systems, and can be extremely costly. Furthermore, the 6-degrees of freedom ("DOR") control of magnetic devices afforded by many magnetic guidance systems, which enables total robotic guidance of device introduction, navigation, orientation, and activation within the body, may not be required for all use-cases. For example, navigation of an electrophysiology catheter into the heart may be performed manually, after which there is only a need for improved maintenance of contact force or more controlled mapping.

Improvements are needed.
US 2015/243425 A1 describes a novel apparatus and method to aggregate and displace a plurality of magneto-responsive entities (steerable self-propelled entities or SSPEs) in three dimensions using time-multiplexing. The apparatus for controlling aggregation of SSPEs in a body comprises at least three sets of magnetic field sources arranged in three axes for generating a controlled magnetic field and a controller connected to at least one of said magnetic field sources to create a three dimensional convergence point. The method for aggregating the entities can comprise using a first set and a second set of said magnetic field sources to generate opposed magnetic field gradients in each said set to cause aggregation of said magneto-responsive entities in two axes and wherein the controller is configured to reverse a direction of said magnetic field gradient in a third set of magnetic field sources in a third axis according to a first pre-determined program.

### SUMMARY

The invention is defined in appended independent claim 1, further embodiments are described in the dependent claims. The methods disclosed herein are not claimed but are useful for the understanding of the invention.

Disclosed herein are magnetic navigation systems and methods for applying a magnetic field to an operating region within the body. One general aspect includes a magnetic navigation system for applying a magnetic field to an operating region within the body. The magnetic navigation system also includes a first magnet assembly configured for introduction into a target location of a body; and a second magnet assembly disposed opposite the first magnet assembly, where the first and second magnet assemblies are configured for applying a magnetic field to an operating region between the magnet assemblies. Other embodiments of this aspect include corresponding computer systems, apparatus, and computer programs recorded on one or more computer storage devices, each configured to perform the actions of the methods.

One general aspect includes a system for generating a magnetic field. Other embodiments of this aspect include corresponding computer systems, apparatus, and computer programs recorded on one or more computer storage devices, each configured to perform the actions of the methods.

One general aspect includes a magnetic field source for generating a magnetic field. Other embodiments of this aspect include corresponding computer systems, apparatus, and computer programs recorded on one or more computer storage devices, each configured to perform the actions of the methods.

One general aspect includes a magnetic navigation system for applying a magnetic field to an operating region within the body. The magnetic navigation system also includes a first magnet assembly configured for introduction into a target location of a body; a second magnet assembly disposed opposite the first magnet assembly external to the body, where the first and second magnet assemblies are configured for applying a magnetic field to an operating region between the magnet assemblies; and at least one magnet assembly support for one or more of supporting or moving at least one magnet assembly to change the direction of magnetic field applied to the operating region. Other embodiments of this aspect include corresponding computer systems, apparatus, and computer programs recorded on one or more computer storage devices, each configured to perform the actions of the methods.

One general aspect includes a system for orienting a medical device within an operating region within the body. The system also includes a first magnet assembly configured for introduction into a target location of a body; a second magnet assembly disposed opposite the first magnet assembly, where the first and second magnet assemblies are configured for applying a magnetic field to an operating region between the first and second magnet assemblies; and a magnetically-responsive medical device configured to magnetically interact with the first and second magnet assemblies. Other embodiments of this aspect include corresponding computer systems, apparatus, and computer programs recorded on one or more computer storage devices, each configured to perform the actions of the methods.

One general aspect includes a magnetic navigation system for applying a magnetic field to an operating region within the body. The magnetic navigation system also includes an elongate body configured to be delivered within a body lumen, the elongate body including a distal portion and a proximal portion, where the distal portion may include a first magnet assembly; a second magnet assembly disposed opposite the first magnet assembly external to the body, where the first and second magnet assemblies are configured for applying a magnetic field to an operating region between the first and second magnet assemblies. Other embodiments of this aspect include corresponding computer systems, apparatus, and computer programs recorded on one or more computer storage devices, each configured to perform the actions of the methods.

### BRIEF DESCRIPTION OF THE DRAWINGS

The following drawings show generally, by way of example, but not by way of limitation, various examples discussed in the present disclosure. In the drawings:
FIG. 1 depicts an exemplary robotic magnetic guidance system in accordance with the prior art.
FIG. 2 depicts an exemplary magnetic catheter introduced into a heart via a magnetic guidance system.
FIG. 3 depicts an exemplary catheter body.
FIG. 4 depicts an exemplary catheter tip.
FIG. 5 depicts an exemplary catheter tip.
FIG. 6A depicts an exemplary magnetic catheter introduced into a heart.
FIG. 6B depicts an exemplary magnetic guidance system introduced into an esophagus.
FIG. 6C depicts exemplary movement of the magnetic catheter with the magnetic guidance system.

### DETAILED DESCRIPTION

For certain cardiac mapping and ablation procedures the quality of the mapping and/or ablation depends upon the quality of the contact between the electrode and the cardiac tissue. It is difficult to maintain the desired contact with the moving surface of the heart during the entire cardiac cycle. Typically, relatively stiff medical devices are urged against the surface of the heart with a certain amount of force in an attempt to maintain contact during the entire cardiac cycle.

Early magnetic navigation techniques involved the use of superconducting magnets. While these techniques were, and remain, highly effective, advances in permanent magnetic materials and in the design of permanent magnets, have made it possible to use permanent magnets for magnetic navigation. While the magnetic fields created by superconducting magnets can be readily changing the currents in the superconducting electromagnetic coils, in order to change the magnetic field created by permanent magnets for navigation, it is generally necessary to change the position and/or orientation of the magnetic field applied by permanent magnet by accurately controlling the position and/or orientation of the permanent magnet.

The present disclosure relates to a system for magnetically navigating a medical device in an operating region within the body of a patient, in which a catheter-based device with an array of tunable/adjustable/translatable/rotatable permanent magnets is endoscopically positioned posteriorly adjacent the heart and a corresponding magnet unit is secured externally at a position anteriorly adjacent the heart (i.e. applied to the patient's chest), wherein the system generates a dynamically controllable magnetic field within the operating region of the heart. The magnetic field can then be leveraged by a magnetically-responsive medical device such as a catheter with an orthogonal array of coils that are selectively exercisable to generate precise magnetic moments to cause the device to change orientation within the operating region.

A plurality of field generators can be disposed and energized to generate converging (co-facing) toroidal magnetic fields. Disposition is either coaxial along a common bore axis in the case of two field generators, or at equivalent angles to shared axes (orthogonal disposition) when three or more field generators are applied. Magnetic fields can be generated and focused into higher flux densities toward a convergence plane disposed midway between field generators.

According to another aspect of the disclosure the system includes a support for mounting and/or changing the position and orientation of the external magnet assembly to change the direction of magnetic field applied to the operating region. The support is preferably capable of pivoting the magnet about a first axis that rotates about a second axis perpendicular to the first axis, and translating the magnet, preferably parallel to the second axis.

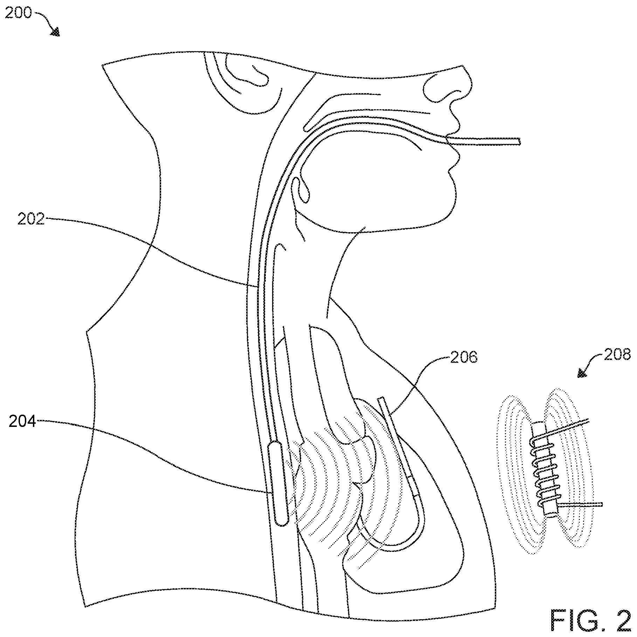

FIG. 2 depicts an overview of the system. FIG. 2 shows a human chest cavity 200 in cut away view. An endo-esophageal magnetic guidance device 204 is placed into a patient's esophagus with a cable 202 for communication with the endo-esophageal magnetic guidance device. A magnetic catheter 206 is placed in the heart. The corresponding magnetic guidance system 208 is placed on the chest. The guidance system interfaces with the endo-esophageal magnetic guidance device to help control the position and orientation of the magnetic catheter 206.

In one embodiment, the present disclosure relates to a magnetic navigation system, and in particular to a system including magnet units comprising a permanent magnet, and a support for controlling the position and orientation of a permanent magnet. The system is adapted for magnetically navigating a medical device in an operating region within the body of a patient. Generally, the system comprises a magnet having a front field projecting from the front of the magnet sufficient to project a magnetic field into the operating region in the patient. The magnet is mounted for movement between a navigation position in which the magnet is located adjacent to the patient with the front of the magnetic generally facing the operating region, and an imaging position in which the magnet is spaced from the patient and the front generally faces away from the operating region.

In one embodiment, the method and apparatus of the present disclosure facilitate the placement of the distal end of a medical device, such as a catheter or micro-catheter, against a target location on a three-dimensional curved surface within a subject body. The method may further provide the feature of determining an external magnetic field to be applied to the medical device for providing a desired estimated contact force against the tissue surface within the subject body. These and other features and advantages will be in part apparent, and in part pointed out hereinafter.

In one embodiment, a method for establishing and estimating the contact force of the tip of a medical device against a tissue surface within a subject body is provided in accordance with the principles of the present disclosure. In one embodiment, the method provides for estimating the contact force of a medical device with a tissue surface such as the heart, through the suitable estimation of the torque applied to the medical device via a magnetic field. While this embodiment is operable with magnetically navigable medical devices, other embodiments of a method in accordance with the present disclosure may be used with medical devices that are guided without magnetic navigation but instead use other control methods for remote navigation such as mechanical actuation, electrostrictive actuation, or hydraulic actuation.

By monitoring the force from the sensor, the remote navigation system may be operated to maintain a satisfactory contact force, either by determining a condition (orientation and position) in which the sensed force is maintained between predetermined minimums and maximums, throughout the entire cardiac cycle, or by dynamically changing the condition (position and orientation) to maintain the sensed force between predetermined minimums and maximums.

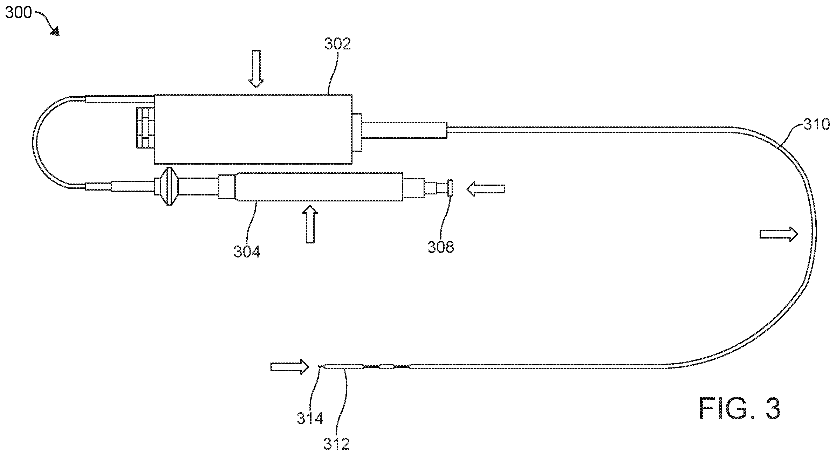

FIGS. 3 - 5 depict an exemplary electrophysiology catheter that is adapted for ablation, mapping, injection, and directional control. In one embodiment, the catheter has a catheter body comprising a handle 302 and a needle deployment/retraction mechanism 304, an intermediate section 310, and a tip section 312 having a tip electrode configured with an omnidirectional distal end and a concentric needle port. The omnidirectional distal end of the tip electrode improves maneuverability and angulation. A dome configuration enables a wide range of tissue contact angles. The concentric needle port provides optimal tissue injection success.

FIG. 3 depicts details of the catheter 300. A needle control handle 302 is attached at the proximal end of a deployment and retraction mechanism 304 for a spring loaded injection needle 314. This mechanism 304 is connected to a fluid connection 308. The catheter intermediate section or shaft 310 connects the needle control handle 302 distally with the catheter tip section 312 and injection needle 314.

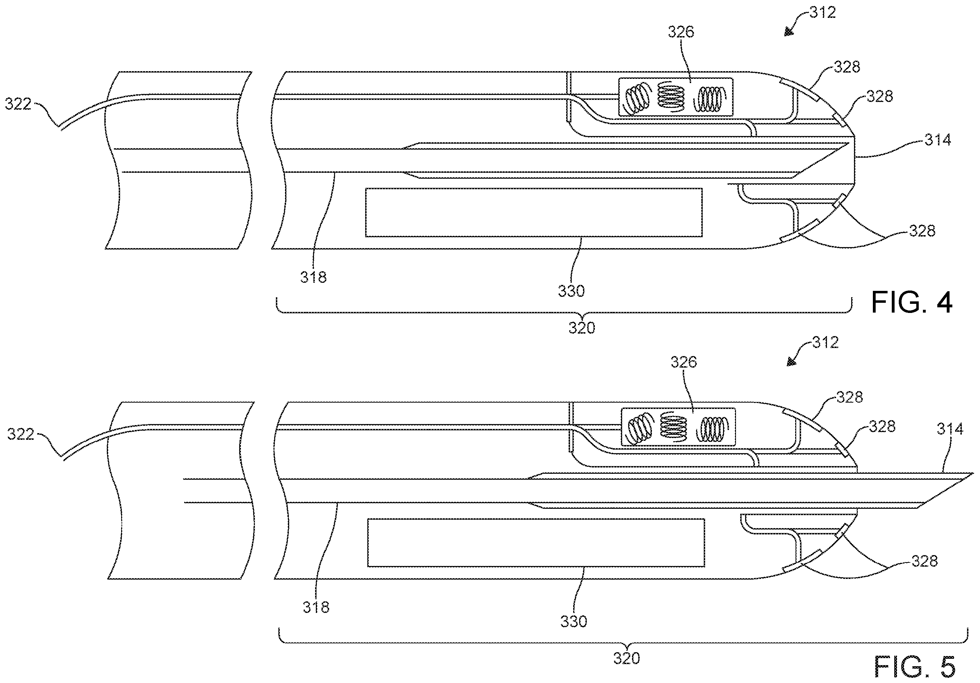

FIGS. 4 and 5 show details of the catheter tip 312. The injection needle 314 is retracted in FIG. 3B and deployed in FIG. 3C. In one embodiment, the tip section 320 houses a position sensor 326 arranged in an integrated configuration, wherein the configuration facilitates a path 318 in the tip section 320 for a component, including an injection needle 314, to extend through the tip section 320 for extension and retraction with reduced stress and friction. The integrated configuration is an efficient use of space that allows the tip section 320 to carry both the position sensor 326 for determining location and orientation of the tip section and the contact sensor or sensors 328 (four are depicted in this embodiment). A magnetic element 330 is also included to allow the magnetic guidance 204 206 system to control the positioning and orientation of the tip section 320. In addition, wiring 322 from the sensors connects to the needle control handle 302. The path 318 defined by the integrated position sensor 326 through the tip section 320 may be generally linear or nonlinear depending on the structure design of the position sensor. For the injection needle 314, the path 318 connects with the concentric needle port whether the path is on axis or off axis with the tip section 320. The catheter also includes a very soft and flexible intermediate section or shaft 310. The catheter tip section 320 may also comprise contact sensors 328 which confirm when the tip section 320 has contacted a body or surface, for instance, the endocardial surface. The injection needle 314 may be off axis or on axis relative to the contact sensor 328

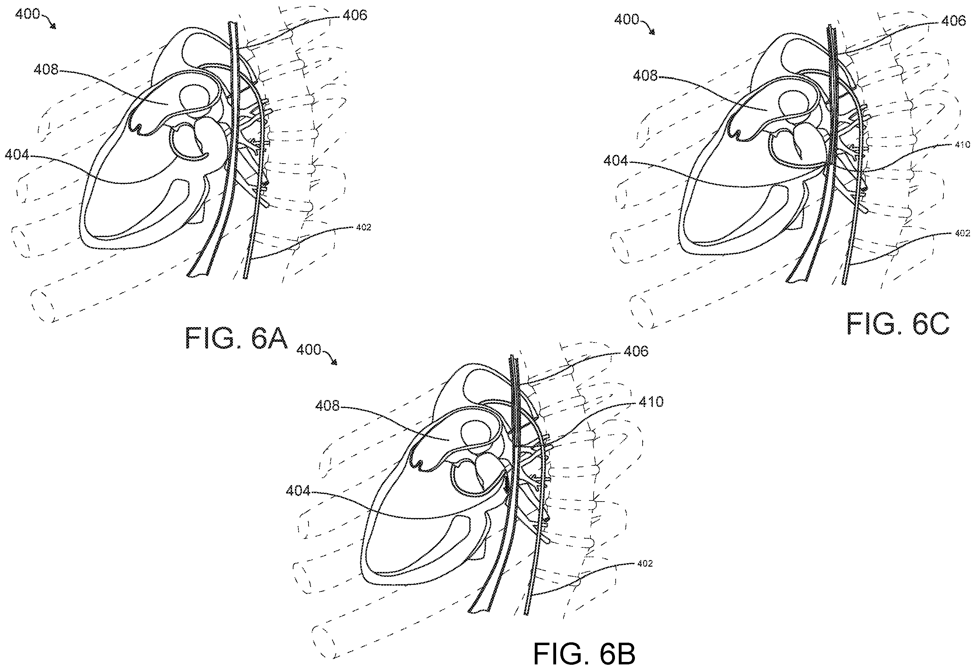

FIGS. 6A-6C depict the catheter in operation. FIG. 6A shows the human heart in 3D section with a catheter 400. The esophagus 406 and the heart 408 are shown most clearly with the skeleton transparent. The magnetic catheter 404 is introduced into a chamber of the heart. The magnetic catheter 404 communicates by the shaft 402 of a catheter. FIG. 6B depicts the introduction of the trans-esophageal catheter 410 with a magnetic element into the esophagus 406. The magnetic element 330 in the magnetic catheter 320 is drawn to the magnetic guidance device 410. The black arrow downward indicates the direction the magnetic catheter 404 and the esophageal magnetic guidance device 410 will travel. FIG. 6C indicates the new location of the magnetic catheter 404 and the magnetic guidance device 410.

Example magnetic navigation systems for applying a magnetic field to an operating region within the body are disclosed herein. Magnetic navigation systems may be endoscopic, minimally-invasive, intraluminal and/or transesophageal. An example system may comprise a first magnet assembly configured for introduction into a bodily passageway (e.g., a body cavity, a vessel, etc., such as an esophagus) and a second magnet assembly disposed opposite the first magnet assembly. The first and second magnet assemblies may be configured for applying a magnetic field to an operating region between the magnet assemblies. As an example, a first device may be placed into the esophagus and a second device applied to the outer surface of the chest, whereby both devices create a magnetic field between them that would include the patient's heart.

In an example embodiment. a magnetic navigation system for applying a magnetic field to an operating region within the body may comprise a first magnet assembly configured for introduction into a target location of a body and a second magnet assembly disposed opposite the first magnet assembly. The first and second magnet assemblies may each comprise at least one magnet configured for applying a magnetic field to an operating region between the magnet assemblies.

A magnetic navigation system for applying a magnetic field to an operating region within the body may comprise a first magnet assembly configured for introduction into a target location of a body and a second magnet assembly disposed opposite the first magnet assembly external to the body. The first and second magnet assemblies may each comprise at least one magnet configured for applying a magnetic field to an operating region between the magnet assemblies.

A magnetic navigation system for applying a magnetic field to an operating region within the body may comprise a first magnet assembly configured for introduction into a target location of a body and a second magnet assembly disposed opposite the first magnet assembly external to the body. The first and second magnet assemblies may be configured for applying a magnetic field to an operating region between the magnet assemblies. The system may further comprise at least one magnet assembly support for supporting at least one magnet assembly.

A magnetic navigation system for applying a magnetic field to an operating region within the body may comprise a first magnet assembly configured for introduction into a target location of a body and a second magnet assembly disposed opposite the first magnet assembly external to the body. The first and second magnet assemblies may be configured for applying a magnetic field to an operating region between the magnet assemblies. The system may further comprise at least one magnet assembly support for supporting and moving at least one magnet assembly to change the direction of magnetic field applied to the operating region.

A system for orienting a medical device within an operating region within the body may comprise a first magnet assembly configured for introduction into a target location of a body and a second magnet assembly disposed opposite the first magnet assembly. The first and second magnet assemblies may be configured for applying a magnetic field to an operating region between the first and second magnet assemblies. The system may further comprise a magnetically-responsive medical device.

A magnetic navigation system for applying a magnetic field to an operating region within the body may comprise an elongate body configured to be delivered within a body lumen, the elongate body including a distal portion and a proximal portion. The distal portion may comprise a first magnet assembly. The system may further comprise a second magnet assembly disposed opposite the first magnet assembly external to the body. The first and second magnet assemblies may be configured for applying a magnetic field to an operating region between the first and second magnet assemblies.

The magnet assemblies may include a positioner for rotating the magnet assemblies about a first axis and pivoting the magnet assemblies about a second axis.

The first and second magnet assemblies may comprise at least one permanent magnet. The magnetic field may be applied with a single permanent magnet, a pair of permanent magnets (for example in a gapped toroid arrangement), a single electromagnetic coil, two or more electromagnetic coils, or a combination thereof.

The first and second magnet assemblies may comprise a compound permanent magnet comprising a plurality of segments of magnetic material.

The system may further comprise a positioner for rotating the magnet about a first axis and pivoting the magnet about a second axis.

The magnet assemblies may be movably mounted on a support for movement about the body, and the magnet assemblies being positioned to project a magnetic field in a direction to orient a magnetically responsive medical device in a selected direction.

The system may further comprise rotating and pivoting each magnet assembly to maintain the magnetic field direction projected by the magnet assemblies in relation to the magnetically-responsive medical device as the magnet assemblies move on the support about an operating region of the body to maintain the magnetically-responsive medical device in a selected orientation as the magnet assemblies move on the support.

The positions of the magnet assemblies may be adjusted as the magnet assemblies move to change the direction of the magnetic field applied by the magnet assemblies to maintain the device in substantially the selected direction despite changes in the distance between the magnet assemblies and the operating region.

The system may further comprise a system controller to control a movement of a magnetically-responsive medical device. The controller may control the positioners of each of the magnet assemblies to change the positions of the magnet assemblies in response to a user-input to apply the magnetic field in the operating region to cause the magnetically responsive device to orient substantially in the selected direction. The controller may control the positioners of each of the magnet assemblies to change the positions of the magnet assemblies as the magnet assemblies move in order to maintain the magnetic field direction. The controller may control the positioners in response to movement of the magnet assemblies, to apply a field whose direction may be determined based upon a user-selected direction and a strength of the field in the operation region.

The first axes of the magnet assemblies may be parallel. Each of the magnet assemblies may also comprise a positioner for controlling the position of the magnet, e.g. for rotating the magnet about a first axis and pivoting the magnet about a second axis, to selectively change the magnetic field applied by each magnet to the operating region in a body on the subject support. In a preferred embodiment, the magnet assemblies, and thus their respective magnets, are mounted on opposite sides of the operating region. The first axis of rotation of each magnet preferably extends through the respective magnet and the operating region, and preferably the first axes of rotation of the magnets are co-linear. The second axes of pivoting of each magnet is preferably perpendicular to the first axis, and also rotates about the first axis.

The first axes of the magnet assemblies may be collinear and extend through the operating region.

The magnet assemblies may each comprise a compound permanent magnet comprising a plurality of segments of magnetic material with differing magnetization directions so that relatively small rotations or pivots change the magnetic field projected by the magnet at a specific point.

The magnet assemblies may be translatable along a first axis that extends radially outwardly from the center of the operating region, may be pivotable about a second axis, generally perpendicular to the first axis that extends through the center of mass of the magnet, and may be rotatable about the first axis.

The system may further comprise changing the magnetic moment of the magnetically-responsive medical device by selectively changing (i.e., via manual manipulation by a user, or a controller) a physical condition of at least one magnet element in the magnetically-responsive medical device to change the orientation of the device with respect to the applied magnetic field.

The system may further comprise applying electrical energy to create temporary magnetic moments in one or more coils in the magnetically-responsive medical device to change the orientation of the magnetically-responsive medical device with respect to the static magnetic field, and orient the magnetically-responsive medical device in a selected direction within the operating region.

A system for generating a magnetic field may comprise at least three magnetic field sources operable to radiate at least three magnetic fields into the anatomical body. Each magnetic field may have a moment different from each moment of each of the other two magnetic fields relative to a fixed point in space.

A magnetic field source for generating a magnetic field may comprise a first coil corresponding to a first magnetic pole and a second coil corresponding to a second magnetic pole. The first magnetic pole may be moveable with respect to said second magnetic pole.

## Claims

1. A magnetic navigation system for applying a magnetic field to an operating region within the body (200), the system comprising:
a first magnet assembly (204) configured for introduction into a target location of a body (200); and
a second magnet assembly (208) disposed opposite the first magnet assembly (204),
wherein the first and second magnet assemblies (204, 208) are configured for applying a magnetic field to an operating region between the magnet assemblies;
**characterized in that** the first and second magnet assemblies (204, 208) each comprise a compound permanent magnet comprising a plurality of segments of magnetic material with differing magnetization directions so that relatively small rotations or pivots change the magnetic field projected by the magnet at a specific point.

2. The system of claim 1 wherein the second magnet assembly (208) is disposed external to the body (200), and wherein the system further comprises:
at least one magnet assembly support for one or more of supporting or moving at least one magnet assembly to change the direction of magnetic field applied to the operating region.

3. The system of claim 2, wherein the positions of the magnet assemblies are adjusted as the magnet assemblies move to change the direction of the magnetic field applied by the magnet assemblies to maintain a device in substantially the selected direction despite changes in the distance between the magnet assemblies and the operating region.

4. The system of claim 1, further comprising:
a magnetically-responsive medical device (206) configured to magnetically interact with the first and second magnet assemblies (204, 208).

5. The system of claim 1 or claim 4, wherein the second magnet assembly (208) is disposed external to the body.

6. The system of claim 4, wherein the magnet assemblies are movably mounted on a support for movement about the body, and the magnet assemblies being positioned to project a magnetic field in a direction to orient a magnetically responsive medical device in a selected direction, and wherein rotating and pivoting each magnet assembly to maintain the magnetic field direction projected by the magnet assemblies in relation to the magnetically-responsive medical device as the magnet assemblies move on the support about an operating region of the body to maintain the magnetically-responsive medical device in a selected orientation as the magnet assemblies move on the support.

7. The system of claim 4, wherein the positions of the magnet assemblies are adjusted as the magnet assemblies move to change the direction of the magnetic field applied by the magnet assemblies to maintain the body in substantially the selected direction despite changes in the distance between the magnet assemblies and the operating region.

8. The system of claim 4, further comprising a system controller to control a movement of the magnetically-responsive medical device (206).

9. The system of claim 8, wherein the controller controls positioners of each of the magnet assemblies to change the positions of the magnet assemblies in response to a user-input to apply the magnetic field in the operating region to cause the magnetically responsive device to orient substantially in the selected direction, or
wherein the controller controls positioners of each of the magnet assemblies to change the positions of the magnet assemblies as the magnet assemblies move in order to maintain the magnetic field direction, or
wherein the controller controls positioners in response to movement of the magnet assemblies, to apply a field whose direction is determined based upon a user-selected direction and a strength of the field in the operation region.

10. The system of claim 4, further comprising changing the magnetic moment of the magnetically-responsive medical device by selectively changing a physical condition of at least one magnet element in the magnetically-responsive medical device to change the orientation of the body with respect to the applied magnetic field.

11. The system of claim 4, further comprising applying electrical energy to create temporary magnetic moments in one or more coils in the magnetically-responsive medical device to change the orientation of the magnetically-responsive medical device with respect to the static magnetic field, and orient the magnetically-responsive medical device in a selected direction within the operating region.

12. The system of claim 1, further comprising:
an elongate body (202) configured to be delivered within a body lumen, the elongate body including a distal portion and a proximal portion, wherein the distal portion comprises the first magnet assembly (204);
wherein the second magnet assembly is disposed external to the body.

13. The system of claim 1, claim 2, claim 4, or claim 12, wherein the magnet assemblies including a positioner for rotating the magnet assemblies about a first axis and pivoting the magnet assemblies about a second axis.

14. The system of claim 1, claim 2, claim 4, or claim 12, wherein the first and second magnet assemblies (204, 208) further comprise a single electromagnetic coil, and/or two or more electromagnetic coils.

15. The system of claim 1, claim 2, claim 4, or claim 12, further comprising a positioner for rotating the magnet assemblies about a first axis and pivoting the magnet assemblies about a second axis.

16. The system of claim 1, claim 2, claim 4, or claim 12, wherein the magnet assemblies are movably mounted on a support for movement about the body (200), and the magnet assemblies being positioned to project a magnetic field in a direction to orient a magnetically responsive medical device (206) in a selected direction.

17. The system of claim 12, wherein the positions of the magnet assemblies are adjusted as the magnet assemblies move to change the direction of the magnetic field applied by the magnet assemblies to maintain the body in substantially the selected direction despite changes in the distance between the magnet assemblies and the operating region.

18. The system of claim 1, claim 2, claim 4, or claim 12, wherein first axes of the magnet assemblies are parallel, or
wherein first axes of the magnet assemblies are collinear and extend through the operating region.

19. The system of claim 1, claim 2, claim 4 or claim 12, wherein the magnet assemblies are translatable along a first axis that extends radially outwardly from the center of the operating region, are pivotable about a second axis, generally perpendicular to the first axis that extends through the center of mass of the magnet assemblies, and are rotatable about the first axis.

## Patentansprüche

1. Magnetisches Navigationssystem zum Anlegen eines Magnetfelds an einen Operationsbereich innerhalb eines Körpers (200), wobei das System umfasst:
eine erste Magnetbaugruppe (204), die zur Einführung in eine Zielstelle des Körpers (200) konfiguriert ist; und
eine zweite Magnetbaugruppe (208), die der ersten Magnetbaugruppe (204) gegenüberliegend angeordnet ist,
wobei die erste und die zweite Magnetbaugruppe (204, 208) konfiguriert sind, um ein Magnetfeld an einen Operationsbereich zwischen den Magnetbaugruppen anzulegen;
**dadurch gekennzeichnet, dass** die erste und die zweite Magnetbaugruppe (204, 208) jeweils einen zusammengesetzten Permanentmagneten umfassen, der eine Vielzahl von Segmenten aus magnetischem Material mit unterschiedlichen Magnetisierungsrichtungen umfasst, sodass relativ kleine Drehungen oder Schwenkungen das von dem Magneten an einem bestimmten Punkt projizierte Magnetfeld ändern.

2. System nach Anspruch 1, wobei die zweite Magnetbaugruppe (208) außerhalb des Körpers (200) angeordnet ist, und wobei das System ferner umfasst:
mindestens einen Magnetbaugruppenträger für eines oder mehreres von Tragen oder Bewegen mindestens einer Magnetbaugruppe, um die Richtung des an den Operationsbereich angelegten Magnetfelds zu ändern.

3. System nach Anspruch 2, wobei die Positionen der Magnetbaugruppen angepasst werden, während sich die Magnetbaugruppe bewegen, um die Richtung des durch die Magnetbaugruppen angelegten Magnetfelds zu ändern, um eine Vorrichtung trotz Änderungen des Abstands zwischen den Magnetbaugruppen und dem Operationsbereich im Wesentlichen in der ausgewählten Richtung zu halten.

4. System nach Anspruch 1, ferner umfassend:
eine magnetisch ansprechbare medizinische Vorrichtung (206), die konfiguriert ist, um mit der ersten und der zweiten Magnetbaugruppe (204, 208) magnetisch zusammenzuwirken.

5. System nach Anspruch 1 oder Anspruch 4, wobei die zweite Magnetbaugruppe (208) außerhalb des Körpers angeordnet ist.

6. System nach Anspruch 4, wobei die Magnetbaugruppen beweglich auf einem Träger für eine Bewegung um den Körper montiert sind, und die Magnetbaugruppen so positioniert sind, dass sie ein Magnetfeld in einer Richtung projizieren, um eine magnetisch ansprechbare medizinische Vorrichtung in einer ausgewählten Richtung auszurichten, und wobei das Drehen und Schwenken jeder Magnetbaugruppe die von den Magnetbaugruppen projizierte Magnetfeldrichtung in Bezug auf die magnetisch ansprechbare medizinische Vorrichtung beibehält, während sich die Magnetbaugruppen auf dem Träger um einen Operationsbereich des Körpers bewegen, um die magnetisch ansprechbare medizinische Vorrichtung in einer ausgewählten Ausrichtung zu halten, während sich die Magnetbaugruppen auf dem Träger bewegen.

7. System nach Anspruch 4, wobei die Positionen der Magnetbaugruppen angepasst werden, während sich die Magnetbaugruppen bewegen, um die Richtung des durch die Magnetbaugruppen angelegten Magnetfelds zu ändern, um den Körper trotz Änderungen des Abstands zwischen den Magnetbaugruppen und dem Operationsbereich im Wesentlichen in der ausgewählten Richtung zu halten.

8. System nach Anspruch 4, ferner umfassend eine Systemsteuerung zum Steuern einer Bewegung der magnetisch ansprechbaren medizinischen Vorrichtung (206).

9. System nach Anspruch 8, wobei die Steuerung Positionierer jeder der Magnetbaugruppen steuert, um die Positionen der Magnetbaugruppen als Reaktion auf eine Benutzereingabe zu ändern, um das Magnetfeld in dem Operationsbereich anzulegen, um zu bewirken, dass sich die magnetisch ansprechbare Vorrichtung im Wesentlichen in der ausgewählten Richtung ausrichtet, oder
wobei die Steuerung Positionierer jeder der Magnetbaugruppen steuert, um die Positionen der Magnetbaugruppen zu ändern, während sich die Magnetbaugruppen bewegen, um die Magnetfeldrichtung beizubehalten, oder
wobei die Steuerung Positionierer als Reaktion auf eine Bewegung der Magnetbaugruppen steuert, um ein Feld anzulegen, dessen Richtung basierend auf einer vom Benutzer ausgewählten Richtung und einer Stärke des Feldes in dem Operationsbereich bestimmt wird.

10. System nach Anspruch 4, ferner umfassend das Ändern des magnetischen Moments der magnetisch ansprechbaren medizinischen Vorrichtung durch selektives Ändern eines physikalischen Zustands mindestens eines Magnetelements in der magnetisch ansprechbaren medizinischen Vorrichtung, um die Ausrichtung des Körpers in Bezug auf das angelegte Magnetfeld zu ändern.

11. System nach Anspruch 4, ferner umfassend das Anlegen elektrischer Energie, um temporäre magnetische Momente in einer oder mehreren Spulen in der magnetisch ansprechbaren medizinischen Vorrichtung zu erzeugen, um die Ausrichtung der magnetisch ansprechbaren medizinischen Vorrichtung in Bezug auf das statische Magnetfeld zu ändern und die magnetisch ansprechbare medizinische Vorrichtung in einer ausgewählten Richtung innerhalb des Operationsbereichs auszurichten.

12. System nach Anspruch 1, ferner umfassend:
einen länglichen Körper (202), der konfiguriert ist, um in ein Körperlumen eingebracht zu werden, wobei der längliche Körper einen distalen Abschnitt und einen proximalen Abschnitt einschließt, wobei der distale Abschnitt die erste Magnetbaugruppe (204) umfasst;
wobei die zweite Magnetbaugruppe außerhalb des Körpers angeordnet ist.

13. System nach Anspruch 1, Anspruch 2, Anspruch 4 oder Anspruch 12, wobei die Magnetbaugruppen einen Positionierer zum Drehen der Magnetbaugruppen um eine erste Achse und zum Schwenken der Magnetbaugruppen um eine zweite Achse einschließen.

14. System nach Anspruch 1, Anspruch 2, Anspruch 4 oder Anspruch 12, wobei die erste und zweite Magnetbaugruppe (204, 208) ferner eine einzelne elektromagnetische Spule und/oder zwei oder mehr elektromagnetische Spulen umfassen.

15. System nach Anspruch 1, Anspruch 2, Anspruch 4 oder Anspruch 12, ferner umfassend einen Positionierer zum Drehen der Magnetbaugruppen um eine erste Achse und zum Schwenken der Magnetbaugruppen um eine zweite Achse.

16. System nach Anspruch 1, Anspruch 2, Anspruch 4 oder Anspruch 12, wobei die Magnetbaugruppen für eine Bewegung um den Körper (200) beweglich an einem Träger montiert sind und die Magnetbaugruppen so positioniert sind, dass sie ein Magnetfeld in eine Richtung projizieren, um eine magnetisch reagierende medizinische Vorrichtung (206) in eine ausgewählte Richtung auszurichten.

17. System nach Anspruch 12, wobei die Positionen der Magnetbaugruppen angepasst werden, während sich die Magnetbaugruppen bewegen, um die Richtung des durch die Magnetbaugruppen angelegten Magnetfelds zu ändern, um den Körper trotz Änderungen des Abstands zwischen den Magnetbaugruppen und dem Operationsbereich im Wesentlichen in der ausgewählten Richtung zu halten.

18. System nach Anspruch 1, Anspruch 2, Anspruch 4 oder Anspruch 12, wobei erste Achsen der Magnetbaugruppen parallel sind, oder
wobei erste Achsen der Magnetbaugruppen kollinear sind und sich durch den Operationsbereich erstrecken.

19. System nach Anspruch 1, Anspruch 2, Anspruch 4 oder Anspruch 12, wobei die Magnetbaugruppen entlang einer ersten Achse, die sich von dem Mittelpunkt des Operationsbereichs radial nach außen erstreckt, verschiebbar sind, um eine zweite Achse, die im Allgemeinen senkrecht zu der ersten Achse verläuft und sich durch den Massenmittelpunkt der Magnetbaugruppen erstreckt, schwenkbar sind, und um die erste Achse drehbar sind.

## Revendications

1. Système de navigation magnétique permettant d'appliquer un champ magnétique à une région de fonctionnement au sein du corps (200), le système comprenant :
un premier ensemble aimant (204) conçu pour introduction dans un emplacement cible d'un corps (200) ; et
un second ensemble aimant (208) disposé à l'opposé du premier ensemble aimant (204),
dans lequel les premier et second ensembles aimant (204, 208) sont conçus pour appliquer un champ magnétique à une région de fonctionnement entre les ensembles aimant ;
**caractérisé en ce que** les premier et second ensembles aimant (204, 208) comprennent chacun un aimant permanent composé comprenant une pluralité de segments de matériau magnétique avec des directions d'aimantation différentes, de sorte que des rotations ou des pivots relativement faibles changent le champ magnétique projeté par l'aimant au niveau d'un point spécifique.

2. Système selon la revendication 1, dans lequel le second ensemble aimant (208) est disposé à l'extérieur du corps (200), et dans lequel le système comprend en outre :
au moins un support d'ensemble aimant pour un ou plusieurs parmi le support ou le déplacement d'au moins un ensemble aimant pour changer la direction du champ magnétique appliqué à la région de fonctionnement.

3. Système selon la revendication 2, dans lequel les positions des ensembles aimant sont ajustées au fur et à mesure que les ensembles aimant se déplacent pour changer la direction du champ magnétique appliqué par les ensembles aimant afin de maintenir un dispositif sensiblement dans la direction sélectionnée malgré des changements dans la distance entre les ensembles aimant et la région de fonctionnement.

4. Système selon la revendication 1, comprenant en outre :
un dispositif médical magnétiquement sensible (206) conçu pour interagir magnétiquement avec les premier et second ensembles aimant (204, 208).

5. Système selon la revendication 1 ou la revendication 4, dans lequel le second ensemble aimant (208) est disposé à l'extérieur du corps.

6. Système selon la revendication 4, dans lequel les ensembles aimant sont montés de manière mobile sur un support pour un déplacement autour du corps, et les ensembles aimant étant positionnés de manière à projeter un champ magnétique dans une direction pour orienter un dispositif médical magnétiquement sensible dans une direction sélectionnée, et dans lequel la rotation et le pivotement de chaque ensemble aimant pour maintenir la direction de champ magnétique projetée par les ensembles aimant par rapport au dispositif médical magnétiquement sensible lorsque les ensembles aimant se déplacent sur le support autour d'une région de fonctionnement du corps afin de maintenir le dispositif médical magnétiquement sensible dans une orientation sélectionnée lorsque les ensembles aimant se déplacent sur le support.

7. Système selon la revendication 4, dans lequel les positions des ensembles aimant sont ajustées au fur et à mesure que les ensembles aimant se déplacent pour changer la direction du champ magnétique appliqué par les ensembles aimant afin de maintenir le corps sensiblement dans la direction sélectionnée malgré des changements dans la distance entre les ensembles aimant et la région de fonctionnement.

8. Système selon la revendication 4, comprenant en outre un dispositif de commande de système pour commander un déplacement du dispositif médical magnétiquement sensible (206).

9. Système selon la revendication 8, dans lequel le dispositif de commande commande des positionneurs de chacun des ensembles aimant pour changer les positions des ensembles aimant en réponse à une entrée utilisateur pour appliquer le champ magnétique dans la région de fonctionnement afin d'amener le dispositif magnétiquement sensible à s'orienter sensiblement dans la direction sélectionnée, ou
dans lequel le dispositif de commande commande des positionneurs de chacun des ensembles aimant pour changer les positions des ensembles aimant au fur et à mesure que les ensembles aimant se déplacent afin de maintenir la direction de champ magnétique, ou
dans lequel le dispositif de commande commande des positionneurs en réponse au déplacement des ensembles aimant, pour appliquer un champ dont la direction est déterminée sur la base d'une direction sélectionnée par l'utilisateur et d'une intensité du champ dans la région de fonctionnement.

10. Système selon la revendication 4, comprenant en outre le changement du moment magnétique du dispositif médical magnétiquement sensible en changeant sélectivement une condition physique d'au moins un élément aimant dans le dispositif médical magnétiquement sensible pour changer l'orientation du corps par rapport au champ magnétique appliqué.

11. Système selon la revendication 4, comprenant en outre l'application d'énergie électrique pour créer des moments magnétiques temporaires dans une ou plusieurs bobines dans le dispositif médical magnétiquement sensible afin de changer l'orientation du dispositif médical magnétiquement sensible par rapport au champ magnétique statique, et orienter le dispositif médical magnétiquement sensible dans une direction sélectionnée au sein de la région de fonctionnement.

12. Système selon la revendication 1, comprenant en outre :
un corps allongé (202) conçu pour être administré au sein d'une lumière corporelle, le corps allongé comportant une partie distale et une partie proximale, dans lequel la partie distale comprend le premier ensemble aimant (204) ;
dans lequel le second ensemble aimant est disposé à l'extérieur du corps.

13. Système selon la revendication 1, la revendication 2, la revendication 4 ou la revendication 12, dans lequel les ensembles aimant comportant un positionneur pour mettre en rotation les ensembles aimant autour d'un premier axe et faire pivoter les ensembles aimants autour d'un second axe.

14. Système selon la revendication 1, la revendication 2, la revendication 4 ou la revendication 12, dans lequel les premier et second ensembles aimant (204, 208) comprennent en outre une seule bobine électromagnétique et/ou deux ou plusieurs bobines électromagnétiques.

15. Système selon la revendication 1, la revendication 2, la revendication 4 ou la revendication 12, comprenant en outre un positionneur pour mettre en rotation les ensembles aimant autour d'un premier axe et faire pivoter les ensembles aimant autour d'un second axe.

16. Système selon la revendication 1, la revendication 2, la revendication 4 ou la revendication 12, dans lequel les ensembles aimant sont montés de manière mobile sur un support pour un déplacement autour du corps (200), et les ensembles aimant étant positionnés pour projeter un champ magnétique dans une direction afin d'orienter un dispositif médical magnétiquement sensible (206) dans une direction sélectionnée.

17. Système selon la revendication 12, dans lequel les positions des ensembles aimant sont ajustées au fur et à mesure que les ensembles aimant se déplacent pour changer la direction du champ magnétique appliqué par les ensembles aimant afin de maintenir le corps sensiblement dans la direction sélectionnée malgré des changements dans la distance entre les ensembles aimant et la région de fonctionnement.

18. Système selon la revendication 1, la revendication 2, la revendication 4 ou la revendication 12, dans lequel des premiers axes des ensembles aimant sont parallèles, ou
dans lequel les premiers axes des ensembles aimant sont colinéaires et s'étendent à travers la région de fonctionnement.

19. Système selon la revendication 1, la revendication 2, la revendication 4 ou la revendication 12, dans lequel les ensembles aimants peuvent être déplacés par translation le long d'un premier axe qui s'étend radialement vers l'extérieur à partir du centre de la région de fonctionnement, peuvent pivoter autour d'un second axe, généralement perpendiculaire au premier axe, qui s'étend à travers le centre de masse des ensembles aimants, et peuvent être en rotation autour du premier axe.
